# EUROPEAN PATENT SPECIFICATION

(11) **EP 1 080 742 B1**
(45) Date of publication and mention of the grant of the patent: **14.12.2005**
(21) Application number: 99910714.7
(22) Date of filing: 26.03.1999
(51) Int. Cl.: A61M 5/142, A61M 5/34

(54) **PLASTIC SYRINGE BARREL AND METHOD OF IMPROVING IT**
SPRITZENZYLINDER AUS KUNSTSTOFF SOWIE VERFAHREN ZU SEINER VERBESSERUNG
CYLINDRE DE SERINGUE EN PLASTIQUE ET PROCEDE SERVANT A L'AMELIORER

(30) Priority: 27.03.1998 JP 8068898
(43) Date of publication of application: 07.03.2001
(73) Proprietor: BRACCO International B.V., 1077 ZX Amsterdam (NL)
(72) Inventor: IIJIMA, Kazumi, Isesaki-shi, 892-2 Gunma (JP)
(74) Representative: von Samson-Himmelstjerna, Friedrich
(86) International application number: PCT/JP1999/001542
(87) International publication number: WO 1999/049914

(56) References cited:
- EP-A- 0 813 884
- JP-A- 9 010 281
- JP-U- 48 001 796
- JP-Z1- 349 313

## Description

The present invention relates to a plastic syringe barrel and a method for improving the same.

### Background Art

A pre-filled plastic syringe, that is, a plastic syringe with relatively large content volume into which a contrast medium is previously injected has been used in recent years. Since the contrast medium has relatively high viscosity, resistance is large in injecting the contrast medium into the body through blood vessels, the spiral cord, and the like. Hence, pressure injection by using a machine is generally performed. In this case, an extension tube or the like is connected to a nozzle portion of the plastic syringe barrel, and the contrast medium is injected under pressure into the body through the extension tube or the like, blood vessels, the spiral cord, and the like. When the contrast medium is injected into the body as described above, high pressure is applied to the inside of the plastic syringe, thereby creating a possibility that the connection portion of the of the plastic syringe barrel and the extension tube or the like becomes disconnected, and thus a luer lock portion for enabling firm connection to the extension tube or the like is formed in the nozzle portion of the plastic syringe barrel. This luer lock portion usually has structure in which a cylindrical space is formed between an inner peripheral surface of an outer cylinder and an outer peripheral surface of an inner cylinder. The extension tube or the like is connected to the nozzle portion of the plastic syringe barrel by screwing a forward end of the extension tube or the like into the luer lock portion thus formed in the cylindrical space.

In this case, however, the connection of these two sometimes becomes loose due to the withdrawal of the screwed extension tube or the like. If injection is performed in loose connection, there is a possibility of the contrast medium leaking out of the connection portion due to high pressure applied during the injection of the contrast medium, which causes a problem.

JP-A-9-10281, on which the preamble of the independent claims of present invention is based on, discloses a plastic syringe barrel in which an outer cylinder and an inner cylinder are formed in a nozzle portion of the plastic syringe barrel. A luer lock portion composed of a cylindrical space is formed between an inner peripheral surface of the outer cylinder and an outer peripheral surface of the inner cylinder.

An object of the present invention is to provide a means capable of firmly and surely connecting the extension tube or the like to the nozzle portion of the plastic syringe barrel and avoiding looseness of the connection.

EP-A-0 813 884 proposes to roughen the surface of the outer surface of the inner cylinder in the male part of a luer lock connection to provide a low axial separation force or removal torque value necessary to remove this medical device to decrease the leakage between the outer surface of the inner cylinder and a mating surface of a medical device connected thereto.

### Disclosure of the Invention

Claim 1 defines a plastic syringe barrel according to the present invention and claim 4 defines a method to improve such a syringe barrel according to the present invention.

In a plastic syringe barrel an outer cylinder and an inner cylinder are formed in a nozzle portion of the plastic syringe barrel and a luer lock portion composed of a cylindrical space is formed between an inner peripheral surface of the outer cylinder and an outer peripheral surface of the inner cylinder, all or part of an inner surface of the luer lock portion is subjected to surface roughening treatment.

In the plastic syringe barrel the inner peripheral surface of the outer cylinder may be subjected to surface roughening treatment. Further, a helically continuous screw thread is formed on the inner peripheral surface of the outer cylinder, and the surface of the screw thread and/or a screw root portion may be subjected to surface roughening treatment. Furthermore, the material of the plastic syringe barrel is cyclic polyolefin resin. Moreover, the surface roughening treatment is performed, for example, by blast treatment.

In a method for improving a plastic syringe barrel in which an outer cylinder and an inner cylinder are formed in a nozzle portion and a luer lock portion composed of a cylindrical space is formed between an inner peripheral surface of the outer cylinder and an outer peripheral surface of the inner cylinder, the connection strength of the luer lock portion is enhanced by forming all or part of an inner surface of the luer lock portion into a surface subjected to surface roughening treatment.

An extension tube or the like having a helical groove is screwed into the luer lock portion and connected thereto. What is connected to the luer lock is not limited to the extension tube, and may be a needle, a three-way cock, or the like. Also, its material is not specially limited.

The plastic syringe barrel "subjected to surface roughening treatment" is not limited to a case where the inner surface of the luer lock portion is directly subjected to surface roughening treatment by subjecting all or part of the inner surface of the luer lock portion to blast treatment or the like. It also includes, for example, a case where in a mold used when the plastic syringe barrel is resin-molded, for example, a mold surface of a portion facing the inner surface of the luer lock portion is formed into a roughened surface and where the inner surface of the luer lock portion is indirectly subjected to surface roughening treatment by transferring the shape of the roughened surface of the mold surface to the inner surface of the luer lock portion at the time of resin molding.

The blast treatment involves blowing hard fine particles such as emery onto the inner surface of the luer lock portion or the mold surface under high pressure to form small protruding and recessed portions thereon, thereby roughening the surface. In this invention, surface roughening treatment is performed by leaving small scratches on all or part of the inner face of the luer lock portion by forming a mold surface of a portion facing the inner surface of the luer lock portion into a roughened surface by being subjected to blast treatment or the like and transferring the shape of the roughened surface of the mold surface to the inner surface of the luer lock portion at the time of resin molding in a mold used when the plastic syringe barrel is resin-molded, or by directly subjecting the inner surface of the luer lock portion to blast treatment or the like. The aforesaid method of subjecting the inner surface of the luer lock portion or the mold surface to surface roughening treatment is not limited to blast treatment, and surface roughening treatment may be performed by forming small scratches on the inner surface of the luer lock portion or the mold surface by the use of a file or the like.

The material of the plastic syringe barrel is, for example, cyclic polyolefin resin, and polycarbonate resin, polymethacrylate resin, polypropylene resin, and the like are also available. The cyclic polyolefin resin is excellent particularly in the effect of being able to firmly and surely connecting the extension tube or the like.

### Brief Description of Drawings

FIG. 1 is an explanatory view of a nozzle portion of a plastic syringe barrel; and
FIG. 2 is an enlarged view showing the structure of a luer lock portion, and the lower half portion of FIG. 2 (a portion lower than a central line shown by an alternate long and short dash line) shows a section.

### Best Mode for Carrying out the Invention

A preferred embodiment of the present invention will be explained below with reference to the attached drawings.

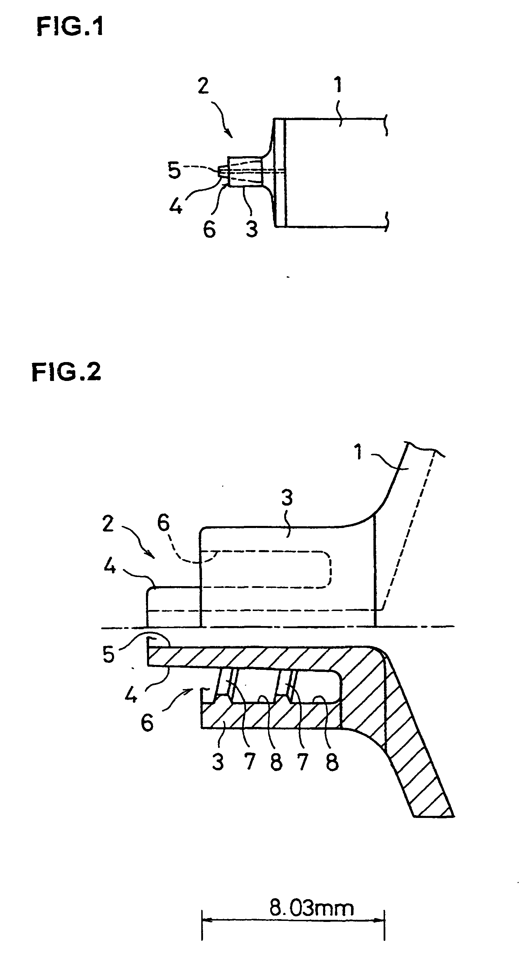

As shown in FIG. 1 and FIG. 2, a nozzle portion 2 is provided at a forward end of a plastic syringe barrel 1. The material of the plastic syringe barrel 1 is, for example, cyclic polyolefin resin. In the nozzle portion 2, an outer cylinder 3 and an inner cylinder 4 are formed, and a liquid such as a contrast medium is filled into the plastic syringe barrel 1. The liquid such as the contrast medium can be injected into the body from within the plastic syringe barrel 1 through a through-hole 5 formed so as to pierce through the middle of the inner cylinder 4.

A luer lock portion 6 composed of a cylindrical space is formed between an inner peripheral surface of the outer cylinder 3 and an outer peripheral surface of the inner cylinder 4. A helically continuous screw thread 7 is formed on the inner peripheral surface of the outer cylinder 3, and a screw root portion 8 is formed in the gap between adjacent ridges of the screw thread 7.

Surface roughening treatment is performed for an inner surface of the luer lock portion 6. The surface roughening treatment is performed by subjecting a mold surface of a portion facing the inner surface of the luer lock portion 6 to blast treatment and transferring the shape of a roughened surface of the mold surface to the inner surface of the luer lock portion 6 at the time of resin molding, for example, in a mold used when the plastic syringe barrel 1 is resin-molded, or by directly subjecting the inner surface of the luer lock portion 6 to blast treatment. The surface roughening treatment may be performed for all of the inner surface of the luer lock portion 6, or may be performed for only part of the inner surface of the luer lock portion 6. For example, in the inner surface of the luer lock portion 6, only the inner peripheral surface of the outer cylinder 3 may be subjected to the surface roughening treatment. Moreover, for example, in the inner surface of the luer lock portion 6, the surfaces of both the helical screw thread 7 and the screw root portion 8 which are formed on the inner peripheral surface of the outer cylinder 3 may be subjected to the surface roughening treatment. Furthermore, the surface roughening treatment may be performed for only the surface of either one of the helical screw thread 7 or the screw root portion 8 formed on the inner peripheral surface of the outer cylinder 3.

In the nozzle portion 2 at the forward end of the plastic syringe barrel 1 structured as above, for example, by screwing a forward end of an extension tube not illustrated into the luer lock portion 6 composed of the cylindrical space, a helical groove formed in a peripheral surface of the extension tube not illustrated can be brought into engagement with the helical screw thread 7 formed on the inner peripheral surface of the outer cylinder 3, whereby the extension tube or the like can be firmly and surely connected to the nozzle portion 2 of the forward end of the plastic syringe barrel 1.

### Industrial Availability

According to the invention in claims 1 to 6, a plastic syringe barrel and an extension tube or the like can be firmly and surely connected, and the connection does not become loose since friction between them is increased by surface roughening treatment. Consequently, there is no fear of a contrast medium leaking out of a connection portion when the contrast medium is injected into the body from within the plastic syringe barrel through blood vessels, the spinal cord, or the like.

## Claims

1. A plastic syringe barrel (1) in which an outer cylinder (3) and an inner cylinder (4) are formed in a nozzle portion (2) of the plastic syringe barrel (1) and in which a luer lock portion (6) composed of a cylindrical space is formed between an inner peripheral surface of the outer cylinder (3) and an outer peripheral surface of the inner cylinder (4),
wherein a helically continuous screw thread (7) is formed on said inner peripheral surface of said outer cylinder (3), **characterised in that**
said inner peripheral surface of said outer cylinder (3) has been subjected to surface roughening treatment.

2. A plastic syringe barrel (1) according to claim 1, wherein the surface of said screw thread (7) and/or a screw root portion (8) has been subjected to surface roughening treatment.

3. The plastic syringe barrel according to claim 1 or 2, wherein the material of the plastic syringe barrel (1) is cyclic polyolefin resin.

4. The plastic syringe barrel (1) according to one of claims 1, 2 or 3, wherein said surface roughening treatment is blast treatment.

5. A method for providing the plastic syringe barrel (1) of claim 1, in which an outer cylinder (3) and an inner cylinder (4) are formed in a nozzle portion (2) and in which a luer lock portion (6) composed of a cylindrical space is formed between an inner peripheral surface of the outer cylinder (3) and an outer peripheral surface of the inner cylinder (4),
wherein a helically continuous screw thread (7) is formed on said inner peripheral surface of said outer cylinder (3), **characterised in that**
said inner peripheral surface of said outer cylinder (3) is subjected to surface roughening treatment.

6. The method of claim 5, wherein the surface of said screw thread (7) and/or screw root portion (8) is subjected to surface roughening treatment.

7. The method of claim 5 or 6, wherein the surface is either subjected directly to the surface roughening treatment, or a mold surface defining the surface is subjected to the surface roughening treatment.

8. The method of any one of claims 5, 6 and 7, wherein said plastic syringe barrel (1) is formed from cyclic polyolefin resin.

9. The method of any one of claims 5, 6, 7 and 8, wherein said surface roughening treatment is performed as a blast treatment.

## Patentansprüche

1. Spritzenzylinder (1) aus Kunststoff, bei welchem ein äußerer Zylinder (3) und ein innerer Zylinder (4) in einem Düsenabschnitt (2) des Spritzenzylinders (1) aus Kunststoff ausgebildet sind, und bei welchem ein Luer-Lock-Abschnitt (6), der aus einem zylindrischen Zwischenraum gebildet wird, zwischen einer inneren Umfangsfläche des äußeren Zylinders (3) und einer äußeren Umfangsfläche des inneren Zylinders (4) ausgebildet ist,
wobei ein spiralförmiges, fortlaufendes Schraubgewinde (7) an der inneren Umfangsfläche des äußeren Zylinders (3) ausgebildet ist, **dadurch gekennzeichnet, daß**
die innere Umfangsfläche des äußeren Zylinders (3) zur Aufrauhung Oberflächen behandelt ist.

2. Spritzenzylinder (1) gemäß Anspruch 1, bei welchem die Oberfläche des Schraubgewindes (7) und/oder ein Gewindegrundbereich (8) zum Aufrauhen Oberflächen behandelt ist.

3. Spritzenzylinder nach Anspruch 1 oder 2, bei welchem der Werkstoff des Spritzenzylinders (1) aus Kunststoff ein zyklisches Polyolefinharz ist.

4. Spritzenzylinder (1) gemäß einem der Ansprüche 1, 2 oder 3, bei welchem die Oberflächenbehandlung zum Aufrauhen eine Strahlbehandlung ist.

5. Verfahren zum Bereitstellen des Spritzenzylinders (1) gemäß Anspruch 1, bei welchem ein äußerer Zylinder (3) und ein innerer Zylinder (4) in einem Düsenabschnitt (2) des Spritzenzylinders (1) aus Kunststoff ausgebildet sind, und bei welchem ein Luer-Lock-Abschnitt (6), der aus einem zylindrischen Zwischenraum gebildet wird, zwischen einer inneren Umfangsfläche des äußeren Zylinders (3) und einer äußeren Umfangsfläche des inneren Zylinders (4) ausgebildet ist,
wobei ein spiralförmiges, fortlaufendes Schraubgewinde (7) an der inneren Umfangsfläche des äußeren Zylinders (3) ausgebildet ist, **dadurch gekennzeichnet, daß**
die innere Umfangsfläche des äußeren Zylinders (3) zur Aufrauhung Oberflächen behandelt ist.

6. Verfahren nach Anspruch 5, bei welchem die Oberfläche des Schraubgewindes (7) und/oder ein Gewindegrundbereich (8) zum Aufrauhen Oberflächen behandelt ist.

7. Verfahren nach Anspruch 5 oder 6, bei welchem die Oberfläche zum Aufrauhen entweder direkt Oberflächen behandelt ist oder eine Formoberfläche, welche die Oberfläche definiert, zum Aufrauhen Oberflächen behandelt ist.

8. Verfahren nach einem der Ansprüche 5, 6 und 7, bei welchem der Spritzenzylinder (1) aus Kunststoff aus einem zyklischen Polyolefinharz geformt ist.

9. Verfahren nach einem der Ansprüche 5, 6, 7 und 8, bei welchem die Behandlung zum Aufrauhen der Oberfläche als Strahlbehandlung durchgeführt wird.

## Revendications

1. Cylindre de seringue en plastique (1) dans lequel un cylindre extérieur (3) et un cylindre intérieur (4) sont formés dans une partie de buse (2) du cylindre de seringue en plastique (1) et dans lequel une partie Luer-lock (6) composée d'un espace cylindrique est formée entre une surface périphérique intérieure du cylindre extérieur (3) et une surface périphérique extérieure du cylindre intérieur (4),
dans lequel un filetage de vis hélicoïdalement continu (7) est formé sur ladite surface périphérique intérieure dudit cylindre extérieur (3), **caractérisé en ce que**
ladite surface périphérique intérieure dudit cylindre extérieur (3) a été soumise à un traitement rendant les surfaces rugueuses.

2. Cylindre de seringue en plastique (1) selon la revendication 1, dans lequel la surface dudit filetage de vis (7) et/ou d'une partie de fond de filet de vis (8) a été soumise à un traitement rendant les surfaces rugueuses.

3. Cylindre de seringue en plastique selon la revendication 1 ou 2, dans lequel le matériau du cylindre de seringue en plastique (1) est une résine polyoléfine cyclique.

4. Cylindre de seringue en plastique (1) selon l'une des revendications 1, 2 ou 3, dans lequel ledit traitement rendant les surfaces rugueuses est un traitement par souffle.

5. Procédé destiné à créer un cylindre de seringue en plastique selon la revendication 1, dans lequel un cylindre extérieur (3) et un cylindre intérieur (4) sont formés dans une partie de buse (2) et dans lequel une partie Luer-lock (6) composée d'un espace cylindre est formée entre une surface périphérique intérieure du cylindre extérieur (3) et une surface périphérique extérieure du cylindre intérieur (4),
dans lequel un filetage de vis hélicoïdalement continu (7) est formé sur ladite surface périphérique intérieure dudit cylindre extérieur (3), **caractérisé en ce que**
ladite surface périphérique intérieure dudit cylindre extérieur (3) est soumise à un traitement rendant les surfaces rugueuses.

6. Procédé selon la revendication 5, dans lequel la surface dudit filetage de vis (7) et/ou d'une partie de fond de filet de vis (8) est soumise à un traitement rendant les surfaces rugueuses.

7. Procédé selon la revendication 5 ou 6, dans lequel la surface est soumise directement au traitement rendant les surfaces rugueuses, ou une surface de moulage définissant la surface est soumise au traitement rendant les surfaces rugueuses.

8. Procédé selon l'une quelconque des revendications 5, 6 et 7, dans lequel ledit cylindre de seringue en plastique (1) est formé à partir d'une résine polyoléfine cyclique.

9. Procédé selon l'une quelconque des revendications 5, 6, 7 et 8, dans lequel ledit traitement rendant les surfaces rugueuses est effectué comme un traitement par souffle.
